# EUROPÄISCHE PATENTSCHRIFT

(11) **EP 1 117 831 B1**
(45) Veröffentlichungstag und Bekanntmachung des Hinweises auf die Patenterteilung: **07.05.2008**
(21) Anmeldenummer: 99948892.7
(22) Anmeldetag: 29.09.1999
(51) Int. Cl.: C12Q 1/68

(54) **VERFAHREN ZUR DNS- ODER RNS-SEQUENZIERUNG**
METHOD FOR DNA- OR RNA-SEQUENCING
PROCEDE POUR LA DETERMINATION DE SEQUENCES D'ADN ET D'ARN

(30) Priorität: 30.09.1998 DE 19844931
(43) Veröffentlichungstag der Anmeldung: 25.07.2001
(73) Patentinhaber: Seeger, Stefan, 8702 Zollikon (CH)
(72) Erfinder: SEEGER,Stefan, 9702 Zollikon (CH)
(74) Vertreter: HOFFMANN EITLE
(86) Internationale Anmeldenummer: PCT/EP1999/007209
(87) Internationale Veröffentlichungsnummer: WO 2000/018956

(56) Entgegenhaltungen:
- EP-A- 0 731 173
- WO-A-90/13666
- WO-A-98/33939
- US-A- 5 302 509
- US-A- 5 547 839
- US-A- 5 674 743
- SEEGER, STEFAN: "Single molecule fluorescence. High performance molecular diagnosis and screening" BIOFORUM , 21(4), 179-180,182-185 , April 1998 (1998-04), XP000878834

## Beschreibung

Die Erfindung betrifft ein Verfahren zur Basensequenzierung von Desoxyribonukleinsäure (DNS) oder Ribonukleinsäure (RNS).

Die Basensequenzierung der Desoxyribonukleinsäure (DNS) und der Ribonukleinsäure (RNS) gehören heute zu den wichtigsten Analysetechniken in der Biotechnologie, pharmazeutischen Industrie, Nahrungsmittelindustrie, medizinischen Diagnostik und anderen Anwendungsfeldern. Die Entschlüsselung der Genome der Organismen eröffnet die Möglichkeit zur Diagnose, Therapie und Prävention von Krankheiten wie auch zur gezielten Modifikation des Erbgutes zur Generierung von Organismen mit modifizierten Eigenschaften. Um dieses Potential jedoch nutzen zu können, sind ausreichend schnelle Sequenzierverfahren erforderlich.

Die klassischen Sequenzierverfahren nach Sanger et al (Proceedings of the National Academy of Science, USA, 74,5463-7; 1977) wie auch nach Maxam und Gilbert (Proceedings of the National Academy of Science, USA, 74, 560-564; 1977), die noch heute Grundlage der Standardsequenzierverfahren sind, benötigen zur Sequenzierung von 200 Nukleotiden 1 bis 3 Tage. Dies erscheint beispielsweise für die Aufgabe, den Humangenom mit ungefähr 3·10⁹ Basenpaaren zu sequenzieren, als zu langsam.

Jüngere Ansätze zur Beschleunigung von Sequenzierverfahren konzentrieren sich auf Verfahren, bei denen einzelne Nukleotide fluoreszenzspektroskopisch detektiert werden. Das US-Patent 4 962 037 offenbart ein Sequenzierverfahren, wonach ein komplementärer Nukleinsäurestrang, bei dem an jeder Base ein für die Base charakteristisches Fluoreszenzfarbstoffmolekül kovalent gebunden ist, an einem Einzelstrang synthetisiert wird. Dieses fluoreszenzmarkierte Nukleinsäuremolekül wird an eine Partikeloberfläche gebunden, wobei das einzelne Partikel beispielsweise mit einer Mikroinjektionspipette in einem Flüssigkeitsstrom gehalten wird. Durch Verwendung einer Exonuklease wird dann jede fluoreszenzmarkierte Base sukzessive von dem Nukleinsäurestrang abgespalten, und in dem Flüssigkeitsstrom in den Fokus eines Laserstrahls geleitet, wo nach der Anregung die für die Base spezifische Fluoreszenz nachgewiesen wird. Die Geschwindigkeit dieses Sequenzierverfahrens ist theoretisch lediglich durch die Schneidegeschwindigkeit der Exonuklease begrenzt, so daß von einer Sequenziergeschwindigkeit von 100 bis 1000 Basen/Sekunde ausgegangen wird.

Voraussetzung für das Durchführen des in US 4 962 037 offenbarten Verfahrens ist, daß lediglich ein einzelnes Nukleinsäuremolekül an einem einzelnen Partikel festgehalten wird. Die Manipulation eines einzelnen Partikels mit einem einzelnen Nukleinsäuremolekül ist jedoch technisch sehr aufwendig und hat sich als für praktische Anwendungen nicht geeignet erwiesen. Weiterhin ist die Verwendung einer Exonuklease notwendig, die Farbstoff-markierte Nukleotide abzuspalten vermag. Dies macht die Entwicklung dieser Verfahren komplizierter, und die Verwendung hierfür modifizierter Exonukleasen führt zudem in der Regel zu einer höheren Ungenauigkeit bei der Basensequenzbestimmung.

US 5,547,839 beschreibt ein Sequenzierverfahren, bei dem durch Zugabe eines Nukleotidgemisches sowie einer Polymerase an einem Einzelstrang ein Komplementärstrang aufgebaut wird. In diesem Gemisch befinden sich auch fluoreszierende Polymerisations-Terminatoren, deren Fluoreszenz durch Abrastern der Oberfläche bestimmt werden kann. Hiernach wird das Fluorophor durch Photozerstörung desaktiviert und der Polymerisations-Terminator entfernt. Anschließend wird das Substrat gewaschen, bevor es einem neuen Polymerisationszyklus unterworfen werden kann.

WO 98/33939 beschreibt ein Sequenzierverfahren umfassend das Befestigen eines DNS-Einzelstranges mit einem Ende an einem durch eine optische Pinzette gehaltenen Bead, an dem anderen Ende an einem durch Magnetkraft gehaltenen Bead. An diesem Einzelstrang wird durch Zugabe von Polymerase und unterschiedlich fluoreszenzgelabelten Nukleotiden der Komplementärstrang aufgebaut, wobei die Fluoreszenz der so eingebauten fluoreszierenden Substanz als fluoreszierendes Mikroskopbild in dem evaneszenten Feld eines Anregungslasers detektiert wird.

Es ist daher Aufgabe der Erfindung, Verfahren zur Basensequenzierung von DNS oder RNS bereitzustellen, bei denen die Vorteile bezüglich der hohen Geschwindigkeit eines Sequenzierverfahrens mit Einzelmoleküläetektion, wie es in dem Stand der Technik beschrieben ist, genutzt werden, jedoch gleichzeitig die oben erwähnten Nachteile überwunden werden. Zur Lösung dieser Aufgabe stellt die Erfindung ein erstes Verfahren zur Basensequenzierung von DNS oder RNS bereit, umfassend die Schritte:
(1) Immobilisieren von DNS- oder RNS-Einzelsträngen auf einer Oberfläche in einer Oberflächendichte von ≤ 1 Molekül/µm²;
(2) Fokussieren eines Laserstrahles auf einen einzelnen, immobilisierten Einzelstrang;
(3) Aufbau eines DNS- oder RNS-Komplementärstranges des immobilisierten, fokussierten Einzelstranges durch Zugabe einer Lösung, enthaltend (i) ein Gemisch von Nukleotiden der Basen Adenin, Cytosin, Guanin und Thymin zum Aufbau eines DNS-Komplementärstranges oder ein Gemisch von Nukleotiden der Basen Adenin, Cytosin, Guanin und Uracil zum Aufbau eines RNS- Komplementärstranges und (ii) eine Polymerase, wobei
   3a) mindestens zwei der vier Nukleotide der Basen Adenin, Cytosin, Guanin und Thymin oder mindestens zwei der vier Nukleotide der Basen Adenin, Cytosin, Guanin und Uracil ganz oder teilweise unterschiedlich lumineszenzmarkiert sind,
   3b) jeder Einbau eines lumineszenzmarkierten Nukleotides in den Komplementärstrang mit einem Einzelmoleküldetektor detektiert wird, und
   3c) vor dem Einbau des jeweils nächsten lumineszenzmarkierten Nukleotides das Lumineszenzsignal des vorhergehenden lumineszenzmarkierten Nukleotides gelöscht wird.

Weiterhin stellt die Erfindung ein zweites Verfahren zur Basensequenzierung von DNS oder RNS bereit, umfassend die Schritte:
(1) Immobilisieren von DNS- oder RNS-Einzelsträngen auf einer Oberfläche in einer Oberflächendichte von ≤ 1 Molekül/µm².
(2) Fokussieren eines Laserstrahles auf einen einzelnen, immobilisierten Einzelstrang;
(3') Aufbau eines DNS- oder RNS-Komplementärstranges des immobilisierten, fokussierten Einzelstranges durch aufeinanderfolgende Zugabe von Lösungen, enthaltend jeweils (i) ein Nukleotid der Basen Adenin, Cytosin, Guanin und Thymin zum Aufbau eines DNS-Komplementärstranges oder ein Nukleotid der Basen Adenin, Cytosin, Guanin und Uracil zum Aufbau eines RNS-Komplementärstranges und (ii) eine Polymerase, wobei
   3a') das in der Lösung enthaltende Nukleotid lumineszenzmarkiert ist,
   3b) jeder Einbau eines lumineszenzmarkierten Nukleotides in den Komplementärstrang mit einem Einzelmoleküldetektor detektiert wird,
   3c) nach Detektion eines Einbaus eines lumineszenzmarkierten Nukleotides in den Komplementärstrang das Lumineszenzsignal des eingebauten Nukleotides gelöscht wird, und
   3d') vor der Zugabe der jeweils nächsten Lösung gespült wird.

Der Begriff DNS- oder RNS-Einzelstrang bezeichnet erfindungsgemäß ein nicht hybridisiertes DNS- bzw. RNS-Molekül. Ein solcher Einzelstrang kann durch direkte Isolierung aus einem Organismus einschließlich gentechnischer Verfahren wie auch durch die Behandlung solcher Moleküle mit Restriktionsenzymen erhalten werden. Oligonukleotide, PCR-Produkte und c-DNA zählen zu diesen Einzelsträngen. Die Herstellung der Einzelstränge aus den Doppelsträngen ist dem Fachmann bekannt, beispielsweise aus J.Sambrook et al., Molecular Cloning, 2. Ausgabe, Cold Spring Harbor Laboratory Press, 1989. Die Behandlung mit Restriktionsenzymen kann unmittelbar vor der Immobilisierung durchgeführt werden, was zu einer Immobilisierung von Molekülen mit unterschiedlichen Basensequenzen führt. Die Einzelstränge haben vorzugsweise 5 bis 2000 Basen, besonders bevorzugt 100 bis 1000 Basen. Es kommen jedoch prinzipiell auch Basenlängen von bis zu 100 Kilobasen in Betracht.

In Schritt (1) der erfindungsgemäßen Verfahren werden die DNS- oder RNS-Einzelstränge auf einer Oberfläche immobilisiert. Die Oberfläche ist vorzugsweise die Oberfläche eines planaren Trägers, der die für die unten beschriebene Einzelmoleküldetektion nötige optische Transparenz aufweist. Besonders bevorzugt ist ein Glasträger, insbesondere ein Quarzglasträger. In einer bevorzugten Ausführungsform wird die Oberfläche des Trägers, auf der die Einzelstränge immobilisiert werden, durch Aufbringen eines Langmuir-Blodgett-Films chemisch modifiziert. Besonders bevorzugt ist ein Langmuir-Blodgett-Film eines Cellulosederivates, insbesondere Trimethylsilylethercellulosecinnammoat (TMSCC) und Aminoalkyltrimethylsilylethercellulose (ATMSC).

Die Einzelstränge können adsorptiv, über eine kovalente Bindung wie auch über Fangmoleküle auf der Oberfläche immobilisiert werden. Fangmoleküle sind insbesondere Nukleotid-Oligomere, die auf der Oberfläche immobilisiert sind und die Einzelstränge durch Hybridisierung binden können. Die Immobilisierung des Oligomers auf der Oberfläche kann durch kovalente Bindung an eine chemisch reaktive Gruppe an der Oberfläche oder durch Adsorption erfolgen. Bevorzugt ist insbesondere die Immobilisierung mit der (Strept-)Avidin-Biotin-Technik, wobei das Oligomer mit Biotin derivatisiert ist und an ein auf der Oberfläche immobilisiertes (Strept-)Avidin-Molekül bindet. Die Immobilisierung des (Strept-)Avidin-Moleküls ist nicht beschränkt. In einer bevorzugten Ausführungsform werden die (Strept-)Avidin-Moleküle über einen Langmuir-Blodgett-Film eines Cellulosederivates auf der Oberfläche immobilisiert. Insbesondere bevorzugt ist es, die Oberfläche zunächst mit 1 bis 8 Monolagen Aminoalkyltrimethylsilylethercellulose (ATMSC) und anschließend 1 bis 8 Monolagen Trimethylsilylethercellulosecinnammoat (TMSCC) zu beschichten. Für die kovalente Ankopplung der (Strept-)Avidin-Moleküle werden dann die Cinnamoyl-Gruppen des TMSCC zu Aldehyd-Gruppen oxidiert. Weiterhin ist es erfindungsgemäß bevorzugt, als Fangmoleküle 5'-Amino-modifizierte Oligonukleotide zu verwenden, die direkt an Aldehyd-Gruppen, beispielsweise Aldehyd-Gruppen, die an Langmuir-Blodgett-Filmen in der oben beschriebenen Art und Weise erhalten werden, binden.

Diese Oberflächendichte wird vorzugsweise durch Regulierung der Oberflächendichte kovalenter Bindungspunkte auf der Oberfläche eingestellt. Eine Möglichkeit hierfür bieten photovernetzbare Langmuir-Blodgett-Filme, wie der oben aufgeführte TMSCC-Film, auf denen in Abhängigkeit von der Bestrahlungszeit mit UV-Licht reaktive Gruppen auf der Oberfläche entstehen. Diese reaktiven Gruppen stehen dann für eine kovalente Bindung von DNS- oder RNS-Einzelsträngen, Nukleotid-Oligomeren oder (Strept-)Avidin-Molekülen zur Verfügung. Alternativ läßt sich die Oberflächendichte der Einzelstränge durch die Konzentration der zu immobilisierenden Einzelstränge oder der zu immobilisierenden Oligomere in der Lösung einstellen. Die Konzentration hängt hierbei von der Oberfläche des Trägers wie auch von dem Volumen der Lösung der zu immobilisierenden Einzelstränge bzw. der zu immobilisierenden Oligomere ab.

In Schritt (2) der erfindungsgemäßen Verfahren wird ein Laserstrahl auf einen einzelnen, immobilisierten Einzelstrang fokussiert. Die Wahl des Laserstrahls hängt hierbei von der verwendeten Lumineszenzmarkierung der Nukleotidbasen ab, die weiter unten beschrieben wird. Zum Fokussieren des Laserstrahls auf den immobilisierten Einzelstrang in Schritt (2) wird vorzugsweise so vorgegangen, daß (a) ein lumineszenzmarkiertes Nukleotid-Oligomer mit dem Einzelstrang hybridisiert wird, (b) die Position des hybridisierten Nukleotid-Oligomers durch Abscannen der Oberfläche, auf der der Einzelstrang immobilisiert ist, mit einem Laserstrahl bestimmt wird, und (c) das Lumineszenzsignal des hybridisierten Nukleotid-Oligomers anschließend gelöscht wird. Der Schritt (a) kann sowohl vor wie auch nach der Immobilisierung des Einzelstranges durchgeführt werden. Hierbei werden die Lumineszenzmarkierung des Nukleotid-Oligomers und der Laserstrahl so gewählt, daß in Schritt (b) die Lumineszenzmarkierung zur Lumineszenz angeregt wird. Das Abscannen der Oberfläche mit dem Laserstrahl in Schritt (b) kann mit herkömmlichen Raster- bzw. Scanvorrichtungen, wie sie beispielsweise in Laserscanningmikroskopen eingesetzt werden, durchgeführt werden. Das Löschen des Lumineszenzsignals in Schritt (c) kann entweder durch Abspalten der Lumineszenzmarkierung, insbesondere Photoabspaltung, oder durch Photobleaching erfolgen.

In Schritt (3) des ersten erfindungsgemäßen Verfahrens wird ein DNS- oder RNS-Komplementärstrang des immobilisierten, fokussierten Einzelstranges durch Zugabe einer Lösung, enthaltend (i) ein Gemisch von Nukleotiden der Basen Adenin, Cytosin, Guanin und Thymin zum Aufbau eines DNS-Komplementärstranges oder ein Gemisch von Nukleotiden der Basen Adenin, Cytosin, Guanin und Uracil zum Aufbau eines RNS- Komplementärstranges und (ii) eine Polymerase aufgebaut. Alternativ könnte auch der Aufbau eines Polymers der Basen Adenin, Cytosin, Guanin und Thymin bzw. der Basen Adenin, Cytosin, Guanin und Uracil zu synthetischer Nukleinsäure, d.h. solchen Nukleinsäuren, die kein Phosphatrückgrat, sondern beispielsweise ein Peptidrückgrat aufweisen (Peptidnukleinsäuren), durchgeführt werden. Erfindungsgemäß sind mindestens zwei der vier Nukleotide der Basen Adenin, Cytosin, Guanin und Thymin oder mindestens zwei der vier Nukleotide der Basen Adenin, Cytosin, Guanin und Uracil ganz oder teilweise unterschiedlich lumineszenzmarkiert. Insbesondere bevorzugt ist es, daß alle vier Basen unterschiedliche Lumineszenzmarkierungen aufweisen. Dies erlaubt die Bestimmung einer Basensequenz bei lediglich einfachem Aufbau eines Komplementärstranges. Werden lediglich zwei der vier Basen unterschiedlich lumineszenzmarkiert, so muß der Aufbau fünfmal wiederholt werden, um eine komplette Sequenz zu erhalten, wobei bei jeder Wiederholung eine unterschiedliche Kombination von markierten Basen verwendet wird. Bei Verwendung von drei unterschiedlich markierten Basen muß der Aufbau des Komplementärstranges dreimal mit jeweils einer unterschiedlichen Kombination von markierten Basen wiederholt werden.

In Schritt (3') des zweiten erfindungsgemäßen Verfahrens wird ein DNS- oder RNS-Komplementärstrang des immobilisierten, fokussierten Einzelstranges durch aufeinanderfolgende Zugabe von Lösungen, enthaltend jeweils (i) ein Nukleotid der Basen Adenin, Cytosin, Guanin und Thymin zum Aufbau eines DNS-Komplementärstranges oder ein Nukleotid der Basen Adenin, Cytosin, Guanin und Uracil zum Aufbau eines RNS-Komplementärstranges und (ii) eine Polymerase aufgebaut, wobei das in der Lösung enthaltende Nukleotid lumineszenzmarkiert ist. Wiederum könnte alternativ auch der Aufbau eines Polymers der Basen Adenin, Cytosin, Guanin und Thymin bzw. der Basen Adenin, Cytosin, Guanin und Uracil zu synthetischer Nukleinsäure, d.h. solchen Nukleinsäuren, die kein Phosphatrückgrat, sondern beispielsweise ein Peptidrückgrat aufweisen (Peptidnukleinsäuren), durchgeführt werden. Gemäß dem zweiten erfindungsgemäßen Verfahren erfolgt der Aufbau des Komplementärstranges durch aufeinanderfolgende Zugaben der jeweiligen Nukleotid-Lösung, wobei vor der Zugabe der jeweils nächsten Lösung gespült werden muß. Sollte nach Zugabe der Nukleotid-Lösung ein Signal eines Einbaus mit dem Einzelmoleküldetektor erfolgen, so kann somit das Signal einer bestimmten Base zugeordnet werden. Werden beispielsweise Lösungen, enthaltend eine Polymerase sowie jeweils ein Nukleotid der Basen Adenin, Cytosin, Guanin und Thymin, zu dem immobilisierten Einzelstrang gegeben, und wird lediglich bei der Zugabe der zweiten Lösung, d.h. der Cytosin-Lösung, ein Signal detektiert, so ist die entsprechende Base des immobilisierten Einzelstranges Guanin. Wird hingeben bei Zugabe sowohl der zweiten als auch der dritten Lösung ein Signal detektiert, so wird somit die Sequenzfolge Guanin-Cytosin auf dem immobilisierten Einzelstrang gefunden. Durch Wiederholung der Zugabe der entsprechenden Lösungen läßt sich so die Gesamtsequenz des immobilisierten Einzelstranges bestimmen.

Die in der Lösung enthaltende Polymerase katalysiert den Aufbau des Komplementärstranges. Ihre Wahl ist nicht beschränkt, sofern sie den Aufbau des Komplementärstranges mit den Farbstoff-markierten Nukleotiden ermöglicht. Beispiele für die erfindungsgemäß verwendbaren Polymerasen sind native T4-Polymerase, native T7-Polymerase, das Klenow-Fragment von E.coli pol I, Exo III, E.coli pol III Holoenzym, die Schlangenvenomphosphodiesterase und Taq-Polymerase.

Jeder durch die Polymerase katalysierte Einbau eines lumineszenzmarkierten Nukleotids in den Komplementärstrang wird erfindungsgemäß mit einem Einzelmoleküldetektor detektiert. Der erfindungsgemäß verwendbare Einzelmoleküldetektor ist nicht beschränkt, sofern er bei gegebenem Detektionsvolumen, gegebener Wellenlänge und Leistung des Laserstrahles und gegebener Lumineszenzmarkierung des Nukleotides die Detektion eines einzelnen lumineszenzmarkierten Nukleotidmoleküls erlaubt. Die Anforderungen an die Empfindlichkeit des Einzelmoleküldetektors steigen hierbei mit zunehmendem Detektionsvolumen und sinkender Leistung des Laserstrahles. Es ist daher insbesondere bevorzugt, das Detektionsvolumen zu minimieren, indem man den Laserstrahl beugungsbegrenzt fokussiert.

Zur Anregung der Lumineszenzmarkierung wird Laserlicht mit einer Wellenlänge von 600 nm und mehr bevorzugt, um das Auftreten von Streulicht zu minimieren. Insbesondere sind Halbleiterlaser in diesem Wellenlängenbereich aus Kostengründen für das erfindungsgemäße Verfahren bevorzugt. Erfolgt die Detektion über eine Fluoreszenzlebensdauer-Messung, so ist der erfindungsgemäß verwendete Laser moduliert, vorzugsweise gepulst.

Die Lumineszenzmarkierung wird auf das verwendete Laserlicht wie auch den verwendeten Einzelmoleküldetektor abgestimmt. Erfindungsgemäß werden bevorzugt Fluorophore als Lumineszenzmarkierung verwendet. Ein geeigneter Satz an Fluorophoren (d.h. ein Fluorophor pro markiertes Nukleotid) wird abhängig von der Art der Detektion gewählt. Zu unterscheiden ist hierbei zwischen der Detektion der Farbe (d.h. der Wellenlänge der emittierten Photonen) und der Detektion der Fluoreszenzlebensdauer des Fluorophors. Beispiele für Farbstoffsätze zur Detektion der Fluoreszenzlebensdauer sind beschrieben in S. Seeger et al., Ber Bunsenges. Physikal. Chem. 97, 1542-1548 (1993) sowie M. Sauer et al., J. Fluorescence 3, 131-139 (1993); Beispiele für Farbstoffsätze zur Detektion der Farbe sind beschrieben in L.M. Smith et al., Nature 312, 674-670 (1989) sowie J.M: Prober et al., Science 238, 336-341 (1987). Beispielsweise können bei Detektion der Fluoreszenzlebensdauer die in Sauer et al., J.Fluor.5, 247-254, 1995 offenbarten Farbstoffe JA22, JA66, JA51-DS sowie der Cynaninfarbstoff Cy5 (Amersham Pharmacia Biotech, Uppsala, Schweden) verwendet werden; bei Detektion der Farbe können die Farbstoffe FAM, JOE, TAMRA und ROX (Applied Biosystems, Foster City, CA, USA) Anwendung finden. Während im ersten erfindungsgemäßen Verfahren die lumineszenzmarkierten Nukleotide mit unterschiedlichen Luminophoren markiert sind, kann im zweiten erfindungsgemäßen Verfahren für jedes Nukleotid der Farbstoff identisch sein, da die Unterscheidung der Nukleotide durch die Verschiedenheit der jeweiligen Lösung ermöglicht wird. Im Hinblick auf eine vereinfachte Anregung und Detektion ist diese Vorgehensweise erfindungsgemäß bevorzugt.

Der Einzelmoleküldetektor umfaßt eine Abbildungsoptik, eine Einheit, die bei Auftreffen von Photonen ein elektrisches Signal erzeugt sowie einen Computer samt Software zur Auswertung der elektrischen Signale. Die Abbildungsoptik ermöglicht vorzugsweise eine zu dem Fokus des Laserstrahles konfokale Abbildung der emittierten Photonen auf die Einheit zur Erzeugung eines elektrischen Signals. Diese Einheit ist vorzugsweise eine Photodiode, insbesondere bevorzugt eine Einzelphotonenzähl-Avalanche-Photodiode. Alternativ kann auch ein Photomultiplier oder eine verstärkte CCD-Kamera verwendet werden. Insbesondere wird bevorzugt, den erfindungsgemäß verwendbaren Einzelmoleküldetektor derart einzurichten, daß die Detektion erst nach einer bestimmten Zeit nach der Anregung durch den Laser beginnt ("Gating"). Ein erfindungsgemäß verwendbarer Einzelmoleküldetektor ist beschrieben in Löscher et al., Anal.Chem., Band 70, S.3202-3205, 1998. Für eine Farbunterscheidung ist der Einzelmolküldetektor mit entsprechenden Filtern ausgestattet. Für eine Unterscheidung durch Messung der Fluoreszenzlebensdauer wird vorzugsweise ein Detektor verwendet, der im Time-correlated-single photon counting-Modus arbeitet. Weiterhin ist eine schnelle Meßelektronik erforderlich, wie sie beispielsweise bei SL Microtest GmbH, Jena, erhältlich ist.

In einer bevorzugten Ausführungsform weist der Einzelmoleküldetektor eine Autokorrelationsfunktion auf. Die Autokorrelationsfunktion ermöglicht, die Lumineszenz frei diffundierender Moleküle von der Lumineszenz am immobilisierten Nukleinsäurestrang zu unterscheiden und dient somit einer Erhöhung des Signal-Rausch-Verhältnisses.

Erfindungsgemäß wird das detektierte Lumineszenzsignal des eingebauten Nukleotids vor dem Einbau des jeweils nächsten lumineszenzmarkierten Nukleotids gelöscht. Dies kann durch Abspalten der Lumineszenzmarkierung erfolgen, insbesondere durch Photoabspaltung. Dies ist möglich, wenn die Lumineszenzmarkierung mit dem Nukleotid über eine photolabile Gruppe gebunden ist, wie beispielsweise in WO 95/31429 offenbart. Ein kurzer Laserpuls führt dann zur Abspaltung der Lumineszenzmarkierung. Zur Abspaltung wird in der Regel eine von der Wellenlänge des Lasers zur Anregung verschiedene Wellenlänge verwendet. Bevorzugt wird ferner das Lumineszenzsignal durch Photobleaching der Lumineszenzmarkierung des Nukleotides gelöscht. Dies läßt sich beispielsweise durch eine kurzfristige Erhöhung der Laserintensität, d.h. einen kurzen Laserpuls, erreichen.

Somit hängt die Geschwindigkeit, mit der das Lumineszenzsignal gelöscht wird, insbesondere von der Leistung des Laserpulses wie auch der Dauer des Zeitraumes zwischen Detektion und Laserpuls ab. Beide Parameter sind gut kontrollierbar. Zur Sicherstellung, daß das Lumineszenzsignal des eingebauten Nukleotides vor dem Einbau des jeweils nächsten Nukleotids gelöscht wird, werden diese Parameter mit der Aufbaugeschwindigkeit des Komplementärstranges abgestimmt. Die Aufbaugeschwindigkeit des Komplementärstranges läßt sich durch Einstellen der Temperatur der Lösung, die einen maßgeblichen Einfluß auf die Polymeraseaktivität hat, und durch Einstellen der Nukleotidkonzentrationen kontrollieren.

Wenn nicht alle Nukleotide in dem ersten erfindungsgemäßen Verfahren lumineszenzmarkiert sind, läßt sich mit einmaligem Aufbau des Komplementärstranges die Basensequenz des immobilisierten Einzelstranges nicht mit ausreichender Genauigkeit in dem ersten erfindungsggemäßen Verfahren bestimmen. Folglich muß zur Bestimmung der kompletten Sequenz der Aufbau wiederholt werden. Dies wird erfindungsgemäß bevorzugt an dem gleichen Einzelstrang durchgeführt. Hierzu wird der wie oben beschrieben aufgebaute DNS- oder RNS-Komplementärstrang von dem immobilisierten DNS- oder RNS-Einzelstrang durch Temperaturerhöhung abgespalten und der Verfahrensschritt (3) des oben beschriebenen Verfahrens wiederholt. Das erfindungsgemäße Verfahren erlaubt somit, die Sequenzbestimmung an dem identischen Molekül mehrmals durchzuführen. Dies ist selbst bei Verwendung nur lumineszenzmarkierter Nukleotide vorteilhaft, um die Genauigkeit der Sequenzbestimmung zu erhöhen.

Alternativ kann das erfindungsgemäße Verfahren an einem weiteren, auf dem Träger immobilisierten Einzelstrang wiederholt werden. Bei Einzelsträngen mit gleicher Basensequenz führt dies zu einer höheren Genauigkeit der Sequenzbestimmung. Ist die zu sequenzierende Nukleinsäure vor der Immobilisierung mit Restriktionsenzymen behandelt worden, ist so jedoch auch die sukzessive Bestimmung der verschiedenen Bruchstücke des Einzelstranges, die durch diese Behandlung entstehen, möglich. Die Behandlung mit Restriktionsenzymen kann hierbei unmittelbar vor der Immobilisierung in dem gleichen Reaktionsgefäß erfolgen.

### Beispiele

1. Beispiel: Bei der schrittweisen Bindung des Einzelstranges wird für etwa 4 cm² Glasoberfläche 1 ml einer 10⁻⁸ mol/l aminofunktionalisierte Oligonukleotid-Lösung über Nacht in PBS-Puffer inkubiert; dann wird durch Zugabe von 1 ml Lösung des Einzelstranges in Hybridisierungspuffer (10⁻⁸ mol/l ) 1 bis 2 h bei Temp.gradient von 58°C zu 28°C hybridisiert; dann wird das Farbstoff-markierte Oligonukleotid (10⁻⁸ mol/l, 1 ml Hybridisierungspuffer, Temp.grad 28°C zu 4°C), 2 - 3 h gegenhybridisiert. Das ergibt eine Dichte von etwa 1 Molekülkomplex/10 µm².
2. Beispiel: Bei der zweiten Vorgehensweise, nämlich die Hybridisierungsschritte vor der Immobilisierung durchzuführen, werden der Einzelstrang, das aminofunktionalisierte Oligonukleoti, und das Farbstoff-markierte Oligonukleotid zusammen in 1 ml Puffer in Konz. Von je 10⁻⁷ mol/l gemischt; schließlich wird diese Lösung auf 10⁻¹⁰ mol/l verdünnt und zur Immobilisierung verwendet; ergibt ebenfalls eine Dichte von etwa 1 Molekülkomplex/10 µm².
3. Beispiel: Zu einem immobilisierten Einzelstrang wurden 2 µl 10⁻¹¹ M Mononukleotide, markiert mit dem Farbstoff Cy5 (Amersham Pharmacia Biotech AB, Uppsala, Schweden), und 3,5 u P7-Sequenase-Polymerase hinzugefügt. Der Einbau des Nukleotides wurde mit einer Laserleistung von 400 µW und einem Fokusdurchmesser von 2 µm detektiert.

## Patentansprüche

1. Verfahren zur Basensequenzierung von DNS oder RNS, umfassend die Schritte:
(1) Immobilisieren von DNS- oder RNS-Einzelsträngen auf einer Oberfläche in einer Oberflächendichte von ≤ 1 Molekül/µm²;
(2) Fokussieren eines Laserstrahles auf einen einzelnen, immobilisierten Einzelstrang;
(3) Aufbau eines DNS- oder RNS-Komplementärstranges des immobilisierten, fokussierten Einzelstranges durch Zugabe einer Lösung, enthaltend (i) ein Gemisch von Nukleotiden der Basen Adenin, Cytosin, Guanin und Thymin zum Aufbau eines DNS-Komplementärstranges oder ein Gemisch von Nukleotiden der Basen Adenin, Cytosin, Guanin und Uracil zum Aufbau eines RNS-Komplementärstranges und (ii) eine Polymerase,
3a) wobei mindestens zwei der vier Nukleotide der Basen Adenin, Cytosin, Guanin und Thymin oder mindestens zwei der vier Nukleotide der Basen Adenin, Cytosin, Guanin und Uracil ganz oder teilweise unterschiedlich lumineszenzmarkiert sind,
3b) jeder Einbau eines lumineszenzmarkierten Nukleotides in den Komplementärstrang mit einem Einzelmoleküldetektor detektiert wird, und
3c) vor dem Einbau des jeweils nächsten lumineszenzmarkierten Nukleotides das Lumineszenzsignal des vorhergehenden lumineszenzmarkierten Nukleotides gelöscht wird.

2. Verfahren zur Basensequenzierung von DNS oder RNS, umfassend die Schritte:
(1) Immobilisieren von DNS- oder RNS-Einzelsträngen auf einer Oberfläche in einer Oberflächendichte von ≤ 1 Molekül/µm²;
(2) Fokussieren eines Laserstrahles auf einen einzelnen, immobilisierten Einzelstrang;
(3') Aufbau eines DNS- oder RNS-Komplementärstranges des immobilisierten, fokussierten Einzelstranges durch aufeinanderfolgende Zugabe von Lösungen, enthaltend jeweils (i) ein Nukleotid der Basen Adenin, Cytosin, Guanin und Thymin zum Aufbau eines DNS-Komplementärstranges oder ein Nukleotid der Basen Adenin, Cytosin, Guanin und Uracil zum Aufbau eines RNS- Komplementärstranges und (ii) eine Polymerase, wobei
3a') das in der Lösung enthaltende Nukleotid lumineszenzmarkiert ist,
3b) jeder Einbau eines lumineszenzmarkierten Nukleotides in den Komplementärstrang mit einem Einzelmoleküldetektor detektiert wird,
3c) nach Detektion eines Einbaus eines lumineszenzmarkierten Nukleotides in den Komplementärstrang das Lumineszenzsignal des eingebauten Nukleotides gelöscht wird, und
3d') vor der Zugabe der jeweils nächsten Lösung gespült wird.

3. Verfahren gemäß Anspruch 1 oder 2, wobei zur Immobilisierung der DNS- oder RNS-Einzelstränge auf einer Oberfläche in Schritt (1) die Einzelstränge über auf der Oberfläche immobilisierte Nukleotid-Oligomere durch Hybridisierung immobilisiert werden.

4. Verfahren gemäß Anspruch 1, wobei die Oberflächendichte der Einzelstränge durch Regulierung der Oberflächendichte kovalenter Bindungspunkte auf der Oberfläche eingestellt wird.

5. Verfahren gemäß Anspruch 1, wobei die Oberflächendichte der Einzelstränge durch die Konzentration der zu immobilisierenden Einzelstränge oder der zu immobilisierenden Oligomere eingestellt wird.

6. Verfahren gemäß einem der vorhergehenden Ansprüche, wobei zum Fokussieren des Laserstrahls auf den immobilisierten Einzelstrang in Schritt (2)
a) ein lumineszenzmarkiertes Nukleotid-Oligomer mit dem Einzelstrang hybridisiert wird,
b) die Position des hybridisierten Nukleotid - Oligomers durch Abscannen der Oberfläche mit einem Laserstrahl bestimmt wird, und
c) das Lumineszenzsignal des hybridisierten Nukleotid-Oligomers anschließend gelöscht wird.

7. Verfahren gemäß einem der vorhergehenden Ansprüche, wobei jeder Einbau eines lumineszenzmarkierten Nukleotides in den Komplementärstrang in Schritt (3b) über die Fluoreszenzlebensdauer des lumineszenzmarkierten Nukleotides detektiert wird.

8. Verfahren gemäß einem der Ansprüche 1 bis 6, wobei jeder Einbau eines lumineszenzmarkierten Nukleotides in den Komplementärstrang in Schritt (3b) durch die Farbe des lumineszenzmarkierten Nukleotides detektiert wird.

9. Verfahren gemäß einem der vorhergehenden Ansprüche, wobei der Einzelmoleküldetektor in Schritt (3b) eine Autokorrelationsfunktion aufweist.

10. Verfahren gemäß einem der vorhergehenden Ansprüche wobei das Löschen des Lumineszenzsignals in Schritt (3c) durch Abspalten der Lumineszenzmarkierung des Nukleotides erfolgt.

11. Verfahren gemäß einem der Ansprüche 1 bis 8, wobei das Löschen des Lumineszenzsignals in Schritt (3c) durch Photobleaching der Lumineszenzmarkierung des Nukleotides erfolgt.

12. Verfahren gemäß einem der vorhergehenden Ansprüche, wobei das Löschen des Lumineszenzsignals vor dem Einbau der jeweils nächsten Base in Schritt (3c) durch Abstimmung der Parameter Polymeraseaktivität, Laserintensität und Nukleotidkonzentration erfolgt.

13. Verfahren zur Basensequenzierung von DNS oder RNS, wobei der gemäß einem der vorhergehenden Ansprüche aufgebaute DNS- oder RNS-Komplementärstrang von dem immobilisierten DNS- oder RNS-Einzelstrang durch Temperaturerhöhung abgespalten wird und das Verfahren gemäß einem der vorhergehenden Ansprüche an dem gleichen Einzelstrang wiederholt wird.

14. Verfahren zur Basensequenzierung von DNS oder RNS, wobei das Verfahren gemäß einem der vorhergehenden Ansprüche an einem weiteren, auf der Oberfläche immobilisierten DNS- oder RNS-Einzelstrang wiederholt wird.

## Claims

1. Process for base sequencing of DNA or RNA, comprising the steps:
(1) immobilising DNA or RNA single strands on a surface in a surface density of ≤ 1 molecule/µm²;
(2) focussing a laser beam on a single, immobilised single strand;
(3) producing a DNA or RNA complementary strand of the immobilised, focussed single strand by adding a solution containing (i) a mixture of nucleotides of the bases adenine, cytosine, guanine and thymine to produce a DNA complementary strand or a mixture of nucleotides of the bases adenine, cytosine, guanine and uracil to produce an RNA complementary strand and (ii) a polymerase,
3a) wherein at least two of the four nucleotides of the bases adenine, cytosine, guanine and thymine or at least two of the four nucleotides of the bases adenine, cytosine, guanine and uracil are luminescence-labelled completely or partially differently,
3b) each insertion of a luminescence-labelled nucleotide into the complementary strand is detected using a single-molecule detector, and
3c) before the insertion of the particular next luminescence-labelled nucleotide, the luminescence signal of the preceding luminescence-labelled nucleotide is quenched.

2. Process for base sequencing of DNA or RNA, comprising the steps:
(1) immobilising DNA or RNA single strands on a surface in a surface density of ≤ 1 molecule/µm²;
(2) focussing a laser beam on a single, immobilised single strand;
(3') producing a DNA or RNA complementary strand of the immobilised, focussed single strand by sequential addition of solutions containing in each case (i) a nucleotide of the bases adenine, cytosine, guanine and thymine to produce a DNA complementary strand or a nucleotide of the bases adenine, cytosine, guanine and uracil to produce an RNA complementary strand and (ii) a polymerase, wherein
3a') the nucleotide present in the solution is luminescence-labelled,
3b) each insertion of a luminescence-labelled nucleotide into the complementary strand is detected using a single-molecule detector,
3c) after detection of an insertion of a luminescence-labelled nucleotide into the complementary strand, the luminescence signal of the inserted nucleotide is quenched, and
3d') before the addition of the particular next solution, rinsing is carried out.

3. Process according to claim 1 or 2, wherein to immobilise the DNA or RNA single strands on a surface in step (1), the single strands are immobilised by hybridisation via nucleotide oligomers immobilised on the surface.

4. Process according to claim 1, wherein the surface density of the single strands is adjusted by regulating the surface density of covalent binding points on the surface.

5. Process according to claim 1, wherein the surface density of the single strands is adjusted by the concentration of the single strands to be immobilised or of the oligomers to be immobilised.

6. Process according to one of the preceding claims, wherein to focus the laser beam on the immobilised single strand in step (2)
a) a luminescence-labelled nucleotide oligomer is hybridised with the single strand,
b) the position of the hybridised nucleotide oligomer is determined by scanning the surface using a laser beam, and
c) the luminescence signal of the hybridised nucleotide oligomer is then quenched.

7. Process according to one of the preceding claims, wherein each insertion of a luminescence-labelled nucleotide into the complementary strand in step 3b) is detected via the fluorescence lifetime of the luminescence-labelled nucleotide.

8. Process according to one of claims 1 to 6, wherein each insertion of a luminescence-labelled nucleotide into the complementary strand in step (3b) is detected by the colour of the luminescence-labelled nucleotide.

9. Process according to one of the preceding claims, wherein the single-molecule detector in step (3b) has an auto-correlation function.

10. Process according to one of the preceding claims, wherein the quenching of the luminescence signal in step (3c) is effected by cleaving the luminescence-labelling of the nucleotide.

11. Process according to one of claims 1 to 8, wherein the quenching of the luminescence signal in step (3c) is effected by photo-bleaching the luminescence-labelling of the nucleotide.

12. Process according to one of the preceding claims, wherein the quenching of the luminescence signal is effected before the insertion of the particular next base in step (3c) by matching the parameters polymerase activity, laser intensity and nucleotide concentration.

13. Process for base sequencing of DNA or RNA, wherein the DNA or RNA complementary strand produced according to one of the preceding claims is cleaved from the immobilised DNA or RNA single strand by temperature increase and the process according to one of the preceding claims is repeated on the same single strand.

14. Process for base sequencing of DNA or RNA, wherein the process according to one of the preceding claims is repeated on a further DNA or RNA single strand immobilised on the surface.

## Revendications

1. Procédé pour la détermination de séquences d'ADN ou d'ARN, comprenant les étapes suivantes :
(1) immobilisation de brins individuels d'ADN ou d'ARN sur une surface, en une densité de surface ≤ 1 molécule/µm² ;
(2) focalisation d'un rayon laser sur un brin individuel immobilisé ;
(3) construction d'un brin complémentaire d'ADN ou d'ARN du brin individuel focalisé immobilisé, par addition d'une solution, contenant (i) un mélange de nucléotides des bases adénine, cytosine, guanine et thymine, pour construire un brin complémentaire d'ADN ou un mélange de nucléotides des bases adénine, cytosine, guanine et uracile pour construire un brin complémentaire d'ARN et (ii) une polymérase,
3a) où au moins deux des quatre nucléotides des bases adénine, cytosine, guanine et thymine, ou au moins deux des quatre nucléotides des bases adénine, cytosine, guanine et uracile, sont marqués par luminescence, en totalité ou en partie, différemment,
3b) chaque insertion d'un nucléotide marqué par luminescence dans le brin complémentaire est détectée avec un détecteur de la molécule individuelle, et
3c) avant insertion de chaque fois le nucléotide marqué par luminescence, immédiatement le suivant, le signal de luminescence du nucléotide marqué par luminescence précédent est éteint.

2. Procédé de détermination de séquences d'ADN ou d'ARN, comprenant les étapes consistant à :
(1) immobiliser des brins individuels d'ADN ou d'ARN sur une surface, en une densité de surface de ≤ 1 molécule/µm² ;
(2) focalisation d'un rayon laser sur un brin individuel immobilisé ;
(3') construction d'un brin complémentaire d'ADN ou d'ARN du brin individuel focalisé immobilisé, par addition successive de solutions, contenant chacune (i) un nucléotide des bases adénine, cytosine, guanine et thymine, pour construire un brin complémentaire d'ADN, ou un nucléotide des bases adénine, cytosine, guanine et uracile, pour construire un brin complémentaire d'ARN, et (ii) une polymérase, où
3a') le nucléotide contenu dans la solution est marqué par luminescence,
3b) chaque insertion d'un nucléotide marqué par luminescence dans le brin complémentaire est détectée avec un détecteur de la molécule individuelle, et
3c) après détection d'une insertion d'un nucléotide marqué par luminescence dans le brin complémentaire, le signal de luminescence du nucléotide marqué par luminescence inséré est éteint.
3d') avant addition de la solution chaque fois immédiatement la suivante, procéder à un rinçage.

3. Procédé selon la revendication 1 ou 2, où, pour immobiliser les brins individuels d'ADN ou d'ARN sur une surface à l'étape (1), les brins individuels sont immobilisés par des oligomères de nucléotides immobilisés sur la surface, par hybridisation.

4. Procédé selon la revendication 1, la densité de surface des brins individuels étant réglée par régulation de la densité de surface de points de liaison covalents sur la surface.

5. Procédé selon la revendication 1, la densité de surface des brins individuels étant réglée par la concentration des brins individuels à immobiliser, ou des oligomères à immobiliser.

6. Procédé selon l'une des revendications précédentes, où, pour focaliser la liaison laser sur le brin individuel immobilisé, à l'étape (2)
a) un oligomère de nucléotide, marqué par luminescence, est hybridisé avec le brin individuel,
b) la position de l'oligomère de nucléotide hybridisé est déterminée en scannant la surface avec un rayon laser, et
c) le signal de luminescence de l'oligomère de nucléotide hybridisé est ensuite éteint.

7. Procédé selon l'une des revendications précédentes, chaque insertion d'un nucléotide marqué par luminescence dans le brin complémentaire, à l'étape (3b), étant détectée par la durée de vie de fluorescence du nucléotide marqué par luminescence.

8. Procédé selon l'une des revendications 1 à 6, où chaque insertion d'un nucléotide marqué par luminescence dans le brin complémentaire à l'étape (3b) est détectée par la couleur du nucléotide marqué par luminescence.

9. Procédé selon l'une des revendications précédentes, où le détecteur de molécules individuelles à l'étape (3b) présente une fonction d'auto-corrélation.

10. Procédé selon l'une des revendications précédentes, où l'extinction du signal de luminescence à l'étape (3c) se fait par dissociation du marquage par luminescence du nucléotide.

11. Procédé selon l'une des revendications 1 à 8, où l'extinction du signal de luminescence à l'étape (3c) s'effectue par photobleaching du marquage par luminescence du nucléotide.

12. Procédé selon l'une des revendications précédentes, où l'extinction du signal de luminescence s'effectuant avant insertion chaque fois de la base immédiatement la suivante à l'étape (3c) par adaptation des paramètres que sont l'activité polymérase, l'intensité laser et la concentration en nucléotides.

13. Procédé de détermination de séquences avec des bases, d'ADN ou d'ARN, le brin complémentaire d'ADN ou d'ARN, construit selon l'une des revendications précédentes, étant dissocié vis-à-vis du brin individuel d'ADN ou d'ARN immobilisé, en procédant à une augmentation de température, et le procédé selon l'une des revendications précédentes étant répété sur le même brin individuel.

14. Procédé de détermination de séquences, par utilisation de bases, d'ADN ou d'ARN, le procédé selon l'une des revendications précédentes étant répété sur un autre brin individuel d'ADN ou d'ARN, immobilisé sur la surface.
